# EUROPÄISCHE PATENTSCHRIFT

(11) **EP 3 284 441 B1**
(45) Veröffentlichungstag und Bekanntmachung des Hinweises auf die Patenterteilung: **02.10.2019**
(21) Anmeldenummer: 17185974.7
(22) Anmeldetag: 11.08.2017
(51) Int. Cl.: A61C 8/02

(54) **TRANSPLANTAT ZUR BESEITIGUNG EINES KNOCHENDEFEKTES AM KIEFER UND VERFAHREN ZUR HERSTELLUNG DES TRANSPLANTATES**
TRANSPLANT FOR REMOVING A BONE DEFECT ON THE JAW AND METHOD OF MANUFACTURING THE TRANSPLANT
TRANSPLANT PERMETTANT D'ÉLIMINER UN DÉFAUT OSSEUX DE LA MÂCHOIRE ET PROCÉDÉ DE FABRICATION DU TRANSPLANT

(30) Priorität: 15.08.2016 DE 102016115093
(43) Veröffentlichungstag der Anmeldung: 21.02.2018
(73) Patentinhaber: Technische Universität Dresden, 01069 Dresden (DE)
(72) Erfinder: Gedrange, Tomasz, 01219 Dresden (DE); Dominiak, Marzena, 55-040 Tyniec Maly (PL)
(74) Vertreter: Riechelmann & Carlsohn Patentanwälte PartG mbB

(56) Entgegenhaltungen:
- WO-A1-2012/154534
- WO-A1-2013/120478
- WO-A1-2015/132432
- DE-A1-102016 000 236

## Beschreibung

Die Erfindung betrifft ein Transplantat zur Beseitigung eines Knochendefektes am Kiefer eines Patienten und ein Verfahren zur Herstellung des Transplantats. Das Transplantat ist insbesondere für einen prophylaktischen Knochenaufbau vor einer kieferorthopädischen Therapie geeignet.

Zähne benötigen für einen sicheren Halt eine gesunde Knochensubstanz. Der aufgrund einer genetischen- oder altersbedingten Knochenvolumen-Reduktion entstehende Knochenverlust sollte nach Möglichkeit durch Knochenaufbau aus eigenem Knochen oder Knochenersatzmaterial erfolgen. Ein Knochenaufbau ist in den Fällen notwendig, in denen nicht genug eigener Kieferknochen für eigene Zähne oder Implantate zur Verfügung steht. Ein Knochenaufbau ist mit einem chirurgischen Eingriff verbunden. Bei den meisten Verfahren erfolgt die Anlagerung körpereigenen Knochens oder Knochenersatzmaterials auf die zu wenig entwickelte Kieferregion. Insbesondere bei einer Zahnfehlstellung kann sich bei der Diagnostik herausstellen, dass nicht genügend eigener Kieferknochen vorhanden ist. Der Grund dafür ist, dass ein Zahn, der gedreht im Alveolarfortsatz steht, nach der Richtigstellung der Diagonalen mehr Raum im Knochen erfordert. Die Folge ist eine nur dünne abdeckende Knochenlamelle, die in manchen Fällen resorbiert wird und zu Rezessionen führen kann [1]. Unter einer Rezession versteht man den Rückgang der Gingiva und des darunter liegenden Alveolarknochens (knöchernes Zahnfach) mit dem Ergebnis, dass ein Teil der Wurzeloberfläche des Zahnes freiliegt. Aber auch bei gesunden Patienten, die keine kieferorthopädische Therapie gehabt haben, kann eine Knochendehiszenz oder ein anderer Defekt vorhanden sein, der nicht bemerkt wurde.

Die kieferorthopädische Zahnbewegung, insbesondere von Zähnen in der Region der labialen oder lingualen Oberfläche des Alveolarknochens, wird häufig als Ursache von Rezessionen diskutiert [8]. Die Position der unteren Schneidezähne hat eine Schlüsselfunktion für jede orthodontische Behandlungsplanung. Es ist allerdings umstritten, ob kieferorthopädische Zahnbewegungen tatsächlich Gingivarezessionen verursachen können oder, ob sich Gingiva und Knochen an die neue Position der Zähne anpassen. In der Literatur finden sich dazu widersprüchliche Aussagen [30]. Insbesondere in der Knochenregion, in der sich die Schneidezähne befinden, kommt es häufig zur spontanen Bildung von Rezessionen. Problematisch ist dabei vor allem der Unterkieferfrontzahnbereich. Die Formen der Unterkiefer unterliegen einer hohen interindividuellen Variabilität [2]. Die Topographie des Unterkieferfrontzahnbereichs wird durch zahlreiche Faktoren charakterisiert: Form und Länge der Wurzeln, Struktur und Umfang des Alveolarknochens, Attachmentniveau, Charakter der bedeckenden Mukosa. Somit unterliegt eine Zahnbewegung im Unterkiefer gewissen anatomischen Grenzen. Bei zu geringem anteriorem Knochenangebot besteht die Gefahr der Überschreitung dieser anatomischen Grenzen, was zu Knochendefekten, Rezessionen und im schlimmsten Falle zu Zahnverlust führen kann. Die Problematik der Schneidezahnbewegungen in Unterkiefer wurde durch eine Reihe experimenteller [3, 4, 5, 6, 7, 8] und klinischer Studien [9, 10, 11, 12] analysiert. Steiner et al. [7] haben in einem Experiment Schneidezähne durch die labiale Kortikalis bewegt. Nach Behandlung fanden sie ausgeprägte Gingivarezessionen und labialen Knochenverlust. Als Ursache für diese Veränderungen wurde ein Spannungszustand der labialen Gingiva und des bedeckenden Periosts als mögliche Ursache angegeben. Engelking und Zachrissen [5] bewegten dieselben labial stehenden Zähne in ihre ursprüngliche Position zurück. Nach fünf Monaten, wurde weder eine signifikante Neubildung von marginalen Knochen noch eine Reduktion der Gingivarezessionen beobachtet [13]. Der einzige Ausweg für den Wiederaufbau von marginalen Knochen und Gingivarezessionen ist der chirurgische Eingriff.

Bis 1990 wurde ein Knochenaufbauverfahren meistens bei Patienten, die Probleme mit der Lagestabilität von Zahnprothesen hatten, durchgeführt. Durch die hohe Misserfolgsquote bei alleinigem Knochenaufbau einerseits und die Fortschritte in der Implantologie andererseits wird Knochenaufbau fast nur noch bei Patienten in Kombination mit Zahnimplantaten durchgeführt. Zielsetzung ist dort die Schaffung eines ausreichend großen Knochenbetts, um Implantate darin einzusetzen. Ohne Implantate muss von einer vollständigen Resorption des verpflanzten Knochens innerhalb der ersten drei Jahre ausgegangen werden [14, 15]. Auch wenn Einbringverfahren von Knochenaufbaumaterial inzwischen recht standardisiert und mit guten Erfolgsaussichten auf Heilung verbunden sind [16, 17, 18, 19], sind diese auf einen Knochenaufbau für eine implantologische oder regenrative Therapie bei Patienten mit Knochendefekt ausgerichtet [20]. Es wurden auf diese Weise bisher nur vertikale Knochenprobleme gelöst.

Es wurden bisher keine chirurgischen Eingriffe zum Knochenaufbau vor einer kieferorthopädischen Therapie durchgeführt. Ähnliche chirurgische Eingriffe für eine implantologische Therapie richten sich immer nach gewissen Kriterien. Dabei entspricht das Einbringen von Knochenaufbaumaterial in aller Regel einem kleineren chirurgischen Eingriff in der Mundhöhle, der mit geringen Komplikationen verbunden ist. Dieser Eingriff wird möglichst schonend durchgeführt, so dass die postoperative Schwellung häufig vermieden werden kann. Bei Patienten mit vollständiger Zahnreihe in einem Kiefer hat die Zahnposition eine große Auswirkung auf den Alveolarknochen. Im Unterkiefer ist die Form des Kieferknochens sehr different und zusätzlich durch unterschiedliche Austrittorte des Nervus mandibularis begrenzt [22]. Der Nerv darf durch OP-Verfahren und das Anbringen eines Knochenfragments nicht verletzt werden, weil es sonst zu bleibenden Sensibilitätsstörungen kommen kann. Diese Probleme haben dazu geführt, dass es kein Verfahren zum Anbringen von Knochenersatzmaterial für vollbezahnte Patienten gibt.

Ein weiteres Problem bei der Planung und Therapie stellt das Management von Weichgewebe dar. Durch ein geringes Angebot an Weichgewebe oder zu geringen Knochenaufbau ist ein zweiter oder dritter operativer Eingriff notwendig. Auch die zusätzlichen Eingriffe garantieren kein optimales Ergebnis.

Neben Knochenersatz werden für den Knochenaufbau auch körpereigene vorgefertigte Knochenstücke operativ verwendet. Dabei wird das Transplantat an das Transplantatlager angepasst. Falsch angepasster Knochen kann zur Irritation des Weichgewebes und zu Entzündungen führen.

Ein Problem stellt auch die Zahnbewegung in einer so aufgebauten Region [23, 24, 25] dar. Einige Knochenersatzprodukte werden schlecht oder auch gar nicht resorbiert. Beispielsweise sind aus Rinderknochen hergestellte Knochenersatzprodukte bekannt, die nicht zu den resorbierbaren Knochenaufbauprodukten gehören. In vielen Studien hat eine Zahnbewegung in einer solchen Region zu Wurzelresorptionen geführt. ß-TCP, als zum Teil resorbierbares Produkt, verursacht Zahnschädigung. Auch die Anwendung von neuen vollresorbierbaren Knochenersatzstoffen hat nicht zum Durchbruch hinsichtlich der Vermeidung einer Wurzelresorptionen geführt [26].

Die Erfinder haben in eigenen Untersuchungen bereits nachgewiesen, dass Knochenaufbau mit Ersatzstoffen kurzfristig zur Zunahme des Knochenvolumens führt [21]. Diese Volumenzunahme ist in allen Ebenen möglich. Eine prophylaktische Behandlung von Knochenlamellen im Frontzahnbereich ist bisher nicht bekannt.

DE 10 2011 011 191 A1 offenbart ein Verfahren zur Herstellung eines Aufsatzes einer Abdeckvorrichtung für eine Knochendefektstelle. Dabei ist die Aufnahme eines Datensatzes, der die Knochendefektstelle repräsentiert, vorgesehen. Die Aufnahme wird verwendet, um ein dreidimensionales Modell des Knochens zu erstellen, das dann verwendet wird, um ein Modell einer angepassten orthopädischen Prothese zu erstellen und eine Form zu bilden, die auf dem angepassten Modell der Prothese basiert. Die Prothesen werden dann in der Form hergestellt und haben die Größe und Form des kundenspezifischen Modells. Dieses Verfahren ist ein kieferchirurgisches Verfahren, das eine anschließende prothetisch-implantologische Therapie erfordert. DE 10 2016 000 236 A1 beschreibt eine Weiterentwicklung dieses Verfahrens. WO 2012/154534 offenbart ein Verfahren zur patientenspezifischen Herstellung einer porösen Metallprothese, bei dem die Zahnschmelzgrenze nicht berücksichtigt wird.

Aufgabe der Erfindung ist es, die Nachteile nach dem Stand der Technik zu beseitigen. Es soll insbesondere ein Verfahren angegeben werden, das die Herstellung eines individuell angepassten Transplantates ermöglicht. Das hergestellte Transplantat soll die Beseitigung eines Knochendefektes ermöglichen, ohne dass eine anschließende prothetisch-implantologische Therapie erforderlich ist.

Diese Aufgabe wird durch die Merkmale der Ansprüche 1, 11, 14 und 15 gelöst. Zweckmäßige Ausgestaltungen der Erfindungen ergeben sich aus den Merkmalen der Unteransprüche.

Nach Maßgabe der Erfindung ist ein Verfahren zur Herstellung eines Transplantats zur Beseitigung eines Knochendefektes am Kiefer eines Patienten vorgesehen, das die Schritte umfasst:
(a) Anfertigen einer dreidimensionalen Aufnahme des Kiefers des Patienten oder eines Teiles des Kiefers, an dem sich der Kieferknochendefekt befindet;
(b) Anfertigen eines virtuellen Modells des Kiefers oder des Teils des Kiefers, an dem sich der Knochendefekt befindet, anhand der dreidimensionalen Aufnahme unter Erhalt eines virtuellen Kiefermodells;
(c) Anfertigen eines virtuellen Modells des Transplantats auf Basis des virtuellen Kiefermodelles unter Erhalt eines virtuellen Transplantatmodells, wobei das virtuelle Transplantatmodell eine Innenfläche, die dem Kieferbogen des virtuellen Kiefermodells im Bereich des Knochendefektes entspricht und mit der das virtuelle Transplantatmodell an dem virtuellen Kiefermodell im Bereich des Knochendefektes anliegt und eine Längskante aufweist, die der Zahnschmelzgrenze zugewandt ist und die um einen Versatz zum Niveau der Zahnschmelzgrenze beabstandet ausgebildet ist; und
(d) Herstellung des Transplantates anhand des virtuellen Transplantatmodells.

Der Ausdruck "virtuelles Kiefermodell" benennt das virtuelle Modell des Kiefers oder des Teils des Kiefers, und zwar auch dann, wenn es sich nur um den Teil des Kiefers handelt, an dem sich der Knochendefekt, befindet.

Unter Zahnschmelzgrenze (engl. cementoenamel junction, abgekürzt "CEJ") wird die am Zahn ausgebildete Grenze zwischen Zahnschmelz, der die Krone eines Zahns überzieht, und Zahngewebe, das die Wurzeln eines Zahns überzieht, verstanden. Die Zahnschmelzgrenze kann in der dreidimensionalen Aufnahme und/oder dem virtuellen Kiefermodell automatisch oder durch den Anwender des Verfahrens identifiziert werden.

Bei dem Teil des Kiefers, von dem in Schritt (a) eine dreidimensionale Aufnahme gefertigt werden soll, kann es sich beispielsweise um den Unterkiefer, den Kieferknochen des Unterkiefers oder den Alveolarfortsatz des Unterkiefers handeln. Der Kieferknochen weist den Alveolarfortsatz auf. Der Alveolarfortsatz weist den Alveolarknochen auf. Der Teil des Kiefers sollte den Alveolarknochen umfassen. Es kann vorgesehen sein, dass nur vom vorderen Bereich des Kiefers, beispielsweise vom vorderen Bereich des Unterkiefers, des Kieferknochens des Unterkiefers oder des Alveolarfortsatzes des Unterkiefers, eine dreidimensionale Aufnahme gefertigt wird. Unter dem vorderen Bereich werden die außen liegenden Abschnitte des Kiefers oder Teils des Kiefers verstanden, also insbesondere die labial und/oder bukkal liegenden Abschnitte des Kiefers oder des Teils des Kiefers.

Das erfindungsgemäße Verfahren ermöglicht die Herstellung individuell angepasster Transplantate. Die erfindungsgemäßen Transplantate können somit als individuelle Knochenfragmente angesehen werden. Das erfindungsgemäße Transplantat kann dem Patienten implantiert und im unterentwickelten Gebiet, d h. im Bereich des Knochendefektes, am Kiefer des Patienten fixiert werden. So entsteht ein ausreichend breiter Knochen für eine Zahnbewegung.

Es kann vorgesehen sein, dass in Schritt (d) zur Herstellung des Transplantates Knochenmaterial des Patienten oder eines Spenders eingesetzt wird. In diesem Fall ist das erfindungsgemäße Transplantat ein Eigenknochen des Patienten oder ein Knochen eines Spenders. Der benötigte Knochen kann aus verschiedenen Stellen des menschlichen Organismus entnommen werden.

Die Erfindung stellt ein Transplantat zur Verfügung, das einen vollindividualisierten Knochenersatz bildet. Damit wird das technische Problem der Passungenauigkeit beseitigt und eine Zahnbewegung besser planbar. Ferner kann die mit der anatomischen Variabilität verbundene Varianz des Nervenkanalverlaufes auf der dreidimensionalen Aufnahme visualisiert [27, 28, 29] und bei der Planung der Verankerungsstellen des erfindungsgemäßen Transplantats am Kiefer, insbesondere am Unterkiefer, des Patienten mit berücksichtigt werden, so dass eine Gefahr für Zahn- oder Nervenschädigung wesentlich reduziert werden kann.

Die Erfindung ermöglicht einen prophylaktischen Knochenaufbau für eine entsprechende Indikation. Vor Beginn einer kieferorthopädischen Therapie kann der Aufbau des vorhandenen Knochens geplant werden. Das erfindungsgemäße Transplantat ermöglicht es, einen Zahn in das neue Knochenfach, das unter Verwendung des Transplantats erhalten wurde, zu bewegen. Die Erfindung trägt dazu bei, dass der Knochen- und Weichgewebeaufbau nach der Zahnbewegung erhalten bleibt. Durch eine nachfolgende kieferorthopädische Therapie werden die Gewebeverluste deutlich reduziert, so dass kein weiterer Knochenaufbau notwendig wird.

Erfindungsgemäß ist in Schritt (c) vorgesehen, dass das virtuelle Transplantatmodell eine Innenfläche aufweist, die dem Kieferbogen des virtuellen Kiefermodells im Bereich des Knochendefektes entspricht und mit der das virtuelle Transplantatmodell an dem virtuellen Kiefermodell im Bereich des Knochendefektes anliegt. Es kann vorgesehen sein, dass das virtuelle Transplantatmodell in Höhenrichtung nicht über den Kieferknochen des virtuellen Kiefermodells übersteht. Es ist vorgesehen, dass das virtuelle Transplantatmodell eine Längskante aufweist, die um einen Versatz zum Niveau der Zahnschmelzgrenze beabstandet ausgebildet ist. Es kann vorgesehen sein, dass der Versatz zum Niveau der Zahnschmelzgrenze in einem Bereich von 1 mm bis 3 mm, vorzugsweise in einem Bereich von 1 mm bis 2 mm oder in einem Bereich von 2 mm bis 3 mm liegt. Diese Längskante des virtuellen Transplantatmodells ist vorzugsweise die Kante, die der Zahnschmelzgrenze zugewandt ist. Sie kann im Wesentlichen parallel zur Zahnschmelzgrenze verlaufen. Das Transplantatmodell kann eine weitere Längskante aufweisen, die der Zahnschmelzgrenze abgewandt ist. Im Falle eines Unterkiefers erstreckt sich das virtuelle Transplantatmodell zweckmäßigerweise in Richtung der Kinnregion. Die Längskante, die der Zahnschmelzgrenze zugewandt ist, ist im Falle eines Unterkiefers die Oberkante des virtuellen Transplantatmodells. Die Längskante, die der Zahnschmelzgrenze abgewandt ist, ist im Falle eines Unterkiefers die Unterkante des virtuellen Transplantatmodells.

Es kann zusätzlich vorgesehen sein, dass das in Schritt (c) angefertigte virtuelle Transplantatmodell eine Breite aufweist, die der Ausdehnung des Knochendefektes des virtuellen Kiefermodells in Breitenrichtung entspricht. Die Breite des virtuellen Transplantatmodells sollte von der Breite des Knochendefektes im virtuellen Kiefermodell abhängen. Es kann außerdem vorgesehen sein, dass an dem virtuellen Transplantatmodell Verankerungsstellen zur Befestigung des Transplantats an dem Kiefer des Patienten festgelegt sind. Bei den Verankerungsstellen kann es sich um Löcher handeln. Durch die Löcher können Fixierungsmittel geführt werden, die eine Befestigung des in Schritt (d) hergestellten Transplantats am Kiefer des Patienten ermöglichen. Es kann in einer Ausführungsform der Erfindung vorgesehen sein, dass das in Schritt (c) angefertigte virtuelle Transplantatmodell Löcher aufweist, die im Transplantat die Durchführung von Fixierungsmitteln ermöglichen. Vorzugsweise sind die Löcher im virtuellen Transplantatmodell so ausgebildet, dass sie jeweils in einem Zahnzwischenraum des virtuellen Kiefermodells liegen. Bei den Fixierungsmitteln kann es sich um Schrauben, beispielsweise um Titanschrauben handeln. Im Falle von sehr engen Zahnzwischenräumen ist die Verwendung von Titanschrauben bevorzugt. Es kann darüber hinaus vorgesehen sein, dass das in Schritt (c) angefertigte virtuelle Transplantatmodell eine maximale Dicke in einem Bereich von 3 bis 4 mm aufweist.

Vorzugsweise sind die Kanten des virtuellen Transplantatmodells abgerundet. Auf diese Weise kann sichergestellt werden, dass der Übergang zwischen Knochen und Transplantat für eine spätere Translation einfacher ist und eine gute Einheilung und Umbau in eigenen Knochen gesichert ist. Die Labialfläche des virtuellen Transplantatmodells sollte vorzugsweise glatt sein.

Das virtuelle Kiefermodell wird in Schritt (b) auf Basis der in Schritt (a) erhaltenen dreidimensionalen Aufnahme gefertigt. Die dreidimensionale Aufnahme kann mittels eines Röntgenverfahrens oder anderen 3D-Aufnahmetechniken angefertigt werden. Beispielsweise kann die dreidimensionale Aufnahme mittels bildgebender Verfahren wie Computertomographie (abgekürzt "CT"), Magnetresonanztomographie oder dentaler Volumentomographie angefertigt werden, wobei die dentale Volumentomographie (abgekürzt "DVT") besonders bevorzugt ist. Typischerweise werden bei diesen Verfahren zunächst mehrere zweidimensionale Aufnahmen erhalten. Diese Aufnahmen können mittels einer Datenverarbeitungsvorrichtung, beispielsweise einem Computer, zu einer dreidimensionalen Aufnahme zusammengesetzt werden. Die dreidimensionale Aufnahme kann als Volumengrafik, beispielsweise einer aus Voxeln zusammengesetzten Volumengrafik, bereitgestellt werden. Aus einer solchen Volumengrafik können wiederum dreidimensionale Ansichten des Kiefers oder des Teils davon, an dem sich der Knochendefekt befindet, erzeugt werden. Der Begriff "dreidimensionale Aufnahme", wie er in der vorliegenden Erfindung verwendet wird, bezeichnet sowohl die unmittelbar mittels der bildgebenden Verfahren erhaltenen Aufnahmen als auch die daraus berechneten Darstellungen des Kiefers oder des Teils davon, vorausgesetzt, dass die Daten eine dreidimensionale Darstellung des Kiefers oder des Teils davon zulassen. Eine dreidimensionale Aufnahme kann eine 3D-Darstellung des Kiefers oder des Teils davon ermöglichen. Die dreidimensionale Aufnahme ermöglicht die Herstellung des virtuellen Kiefermodells.

Die dreidimensionale Aufnahme kann als medizinischer Datensatz bereitgestellt werden. Ein solcher Datensatz wird auch als medizinischer 3D-Datensatz bezeichnet. Der medizinische Datensatz enthält zweckmäßigerweise nur noch die Daten, die zur Bildung eines virtuellen Modells des Kiefers oder eines Teils davon in Schritt (b) des erfindungsgemäßen Verfahrens erforderlich sind. Der medizinische Datensatz kann die Grundlage für ein virtuelles Modell des Kiefers oder eines Teils davon sein. Es ist jedoch auch möglich, alternativ oder zusätzlich die dreidimensionale Aufnahme unmittelbar, d. h. ohne vorherige Bildung eines medizinischen Datensatzes, zur Bildung des virtuellen Modells des Kiefers oder eines Teils davon einzusetzen.

Die dreidimensionale Aufnahme oder der daraus abgeleitete medizinische Datensatz sind nicht zur Erstellung von dreidimensionalen Modellen einer Knochendefektstelle bestimmt. Die Erfindung sieht somit nicht die Aufnahme eines Datensatzes, der die Knochendefektstelle repräsentiert, vor. Hingegen ist die Anfertigung einer dreidimensionalen Aufnahme des Kiefers oder eines Teils davon, aus der optional ein medizinischer Datensatz abgeleitet wird, in Schritt (a) erforderlich, um in Schritt (d) ein Transplantat anfertigen zu können, das beispielsweise aus einem Knochen mittels Fräsen hergestellt werden kann. Die dreidimensionale Aufnahme oder optional der medizinische Datensatz wird nur für die Planung des Transplantates, d. h. die Schritte (b) und (c) des erfindungsgemäßen Verfahrens, verwendet. Schritt (a) des Verfahrens darf sich somit nicht auf die Knochendefektstelle beschränken.

Das Transplantat kann anhand des virtuellen Transplantatmodells aus Knochenmaterial, beispielsweise Eigenknochen des Patienten oder Knochen eines Spenders, oder Knochenersatzmaterial hergestellt werden. Dazu können zum Beispiel Materialbearbeitungsverfahren wie beispielsweise Fräsen oder Formgebungsverfahren wie Drucken oder Pressen eingesetzt werden.

Nach Maßgabe der Erfindung ist ferner ein Transplantat zur Beseitigung eines Knochendefektes am Kiefer eines Patienten vorgesehen, wobei das Transplantat eine Innenfläche, die dem Kieferbogen des Kiefers oder des Teils des Kiefers, der den Knochendefekt aufweist, entspricht, und eine Längskante aufweist, die der Zahnschmelzgrenze zugewandt ist und die um einen Versatz zum Niveau der Zahnschmelzgrenze beabstandet ausgebildet ist. Weil die Innenfläche des Transplantats dem Kieferbogen des Kiefers im Bereich des Knochendefektes entspricht, kann das Transplantat an dem Kiefer im Bereich des Knochendefektes anliegen. Es kann vorgesehen sein, dass das Transplantat in Höhenrichtung nicht über den Kieferknochen des virtuellen Kiefermodells übersteht. Das Transplantat weist eine Längskante auf, die um einen Versatz zum Niveau der Zahnschmelzgrenze beabstandet ausgebildet ist. Es kann vorgesehen sein, dass der Versatz zum Niveau der Zahnschmelzgrenze in einem Bereich von 1 mm bis 3 mm, vorzugsweise in einem Bereich von 1 mm bis 2 mm oder in einem Bereich von 2 mm bis 3 mm liegt. Im Falle eines Unterkiefers erstreckt sich das Transplantat zweckmäßigerweise in Richtung der Kinnregion. Das Transplantat kann eine Breite aufweisen, die der Ausdehnung des Knochendefektes in Breitenrichtung entspricht. Es kann außerdem vorgesehen sein, dass an dem Transplantat Verankerungsstellen zur Befestigung des Transplantats an dem Kiefer des Patienten festgelegt sind. Bei den Verankerungsstellen kann es sich um Löcher handeln. Durch die Löcher können Fixierungsmittel geführt werden, die eine Befestigung des Transplantats am Kiefer des Patienten ermöglichen. Es kann in einer Ausführungsform der Erfindung vorgesehen sein, dass die Löcher im Transplantat so ausgebildet sind, dass sie jeweils in einem Zahnzwischenraum des Kiefers liegen. Das erfindungsgemäße Transplantat ist vorzugsweise so ausgebildet, dass es im gesunden Bereich des Kiefers befestigbar ist. Der Ausdruck "gesunder Bereich" bezieht sich dabei auf einen Bereich des Kiefers, in dem der Knochendefekt, der mittels des Transplantats beseitigt werden soll, nicht ausgebildet ist. Der gesunde Bereich weist keinen Knochendefekt auf.

Bei den Fixierungsmitteln kann es sich um Schrauben, beispielsweise um Titanschrauben handeln. Im Falle von sehr engen Zahnzwischenräumen ist die Verwendung von Titanschrauben bevorzugt. Es kann darüber hinaus vorgesehen sein, dass das Transplantat, vorzugsweise im dicksten Abschnitt, eine Dicke in einem Bereich von 3 bis 4 mm aufweist. Vorzugsweise sind die Kanten des Transplantats abgerundet. Auf diese Weise kann sichergestellt werden, dass der Übergang zwischen Knochen und Transplantat für eine spätere Translation einfacher ist und eine gute Einheilung und Umbau in eigenen Knochen gesichert ist. Die Labialfläche des Transplantats sollte vorzugsweise glatt sein. Das Transplantat kann aus Knochenmaterial des Patienten oder eines Spenders hergestellt sein. In ersten Fall ist das erfindungsgemäße Transplantat ein Eigenknochen des Patienten. Das erfindungsgemäße Transplantat kann mittels des erfindungsgemäßen Verfahrens hergestellt werden.

Nach Maßgabe der Erfindung ist überdies ein Transplantat vorgesehen, das mittels des erfindungsgemäßen Verfahrens hergestellt worden ist.

Schließlich ist nach Maßgabe der Erfindung eine virtuelle Anordnung vorgesehen, die
(i) ein erstes virtuelles Modell eines Kiefers oder des Teils eines Kiefers, an dem sich ein Knochendefekt befindet; und
(ii) ein zweites virtuelles Modell eines Transplantats,
aufweist, wobei das zweite virtuelle Modell eine Innenfläche, die dem Kieferbogen des ersten virtuellen Modells entspricht und mit der das zweite virtuelle Modell an dem ersten virtuellen Modell im Bereich des Knochendefektes anliegt, und eine Längskante aufweist, die der Zahnschmelzgrenze zugewandt ist und die um einen Versatz zum Niveau der Zahnschmelzgrenze beabstandet ausgebildet ist. Das zweite virtuelle Modell weist eine Längskante auf, die um einen Versatz zum Niveau der Zahnschmelzgrenze des Kiefers beabstandet ausgebildet ist. Das erste virtuelle Modell entspricht dem vorstehend beschriebenen Kiefermodell. Das zweite virtuelle Modell entspricht dem vorstehend beschriebenen Transplantatmodell. Weitere Einzelheiten zu den Merkmalen des ersten und zweiten virtuellen Modells können der Beschreibung des erfindungsgemäßen Verfahrens entnommen werden.

Die Erfindung stellt ein Transplantat bereit, das als Knochenersatzmaterial verwendet werden kann. Das Transplantat ermöglicht einen Knochenaufbau am Kiefer eines Patienten vor einer kieferorthopädischen Therapie. Zur Vorbereitung des Knochenaufbaus durch die Implantation des erfindungsgemäßen Transplantats sollte für eine ausreichend breite befestigte Gingiva gesorgt werden. Das erfindungsgemäße Transplantat wird vorzugsweise im gesunden Bereich des Kiefers befestigt. Der Ausdruck "gesunder Bereich" bezieht sich dabei auf einen Bereich des Kiefers, in dem der Knochendefekt, der mittels des Transplantats beseitigt werden soll, nicht ausgebildet ist. Der gesunde Bereich weist keinen Knochendefekt auf. Die Verwendung des erfindungsgemäßen Transplantates ist insbesondere dann vorteilhaft, wenn der Patient bleibende Zähne in einem Bereich des Kiefers aufweist, in dem der Knochendefekt ausgebildet ist. Durch die Nutzung der bleibenden Zähne des Patienten wird eine Knochenresorption in den Knochendefekt verhindert. Unter "bleibenden Zähnen" werden dabei Zähne verstanden, die bis zur Einheilung des implantierten Transplantats, vorzugsweise bis zum Abschluss einer an die Implantation anschließenden kieferorthopädischen Therapie nicht entfernt werden müssen.

Der Einsatz des erfindungsgemäßen Transplantats vermeidet Korrektureingriffe, insbesondere eine sogenannte Dysgnathie-Operation, beispielsweise die Vorverlagerung des Unterkiefers bei Unterkieferrücklage, da das Transplantat eine größere Zahnbewegung in sagittaler Richtung zulässt. Das Risiko von Zahnverlust nach kieferorthopädischer Therapie reduziert sich.

Die Erfindung wird nachstehend anhand von Ausführungsbeispielen, die die Erfindung nicht einschränken sollen, unter Bezugnahme auf die Zeichnungen näher erläutert. Dabei zeigen
- Fig. 1: eine Querschnittsdarstellung eines Unterkiefers mit einer daran befestigten Ausführungsform eines erfindungsgemäßen Transplantats;
- Fig. 2: eine Draufsicht auf den in Fig. 1 gezeigten Unterkiefer mit daran befestigtem Transplantat;
- Fig. 3: eine frontale Ansicht auf den in Fig. 1 gezeigten Unterkiefer mit daran befestigtem Transplantat; und
- Fig. 4: Röntgenaufnahmen eines Unterkiefers mit eingezeichneten Transplantaten gemäß der Erfindung (durchgehende Linie) und gemäß des Standes der Technik (gestrichelte Linie), wobei Fig. 4a eine Schnittansicht und Fig. 4b eine Draufsicht zeigt).

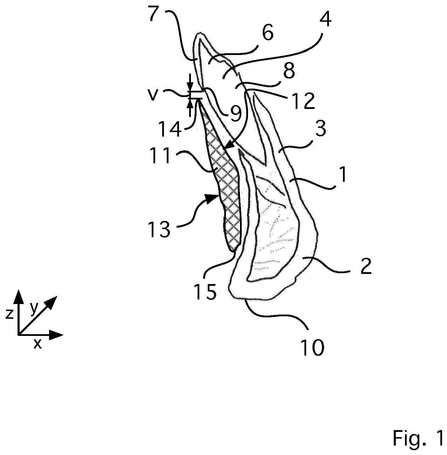

In den Figuren 1 bis 3 ist am Unterkiefer 1 eines Patienten ein Transplant 11 befestigt. Der Unterkiefer 1 weist den Kieferknochen 2 auf, der in seinem Alveolarfortsatz 3 Zähne 4 trägt. Im Bereich des Knochendefektes ist ein Transpantat 11 des Unterkiefers 1 befestigt, und zwar im vorderen Bereich des Unterkiefers 1. Im Kieferknochen 2 sind die Foramina mentalia 5 ausgebildet. Die Zähne 4 weisen Zahnkronen 6, die mit Zahnschmelz 7 bedeckt sind, und Zahnwurzeln 8 auf, die sich in den Alveolarfortsatz 3 erstrecken. Die Grenze zwischen dem mit Zahnschmelz 7 bedeckten Bereich des Zahnes 4, d. h. der Zahnkrone 6, und dem nicht mit Zahnschmelz 7 bedeckten Bereich des Zahnes 4, d. h. den Zahnwurzeln 8, ist die Zahnschmelzgrenze 9 (siehe insbesondere Fig. 1).

Die in den Figuren 1 bis 3 gezeigten Darstellungen des Unterkiefers sind von CT- bzw. DVT-Aufnahmen abgeleitet. Die abgebildete Unterkieferform ist eine interindividuelle anatomische Variable. Diese Darstellungen entsprechen lediglich der groben Form des Unterkiefers eines Patienten.

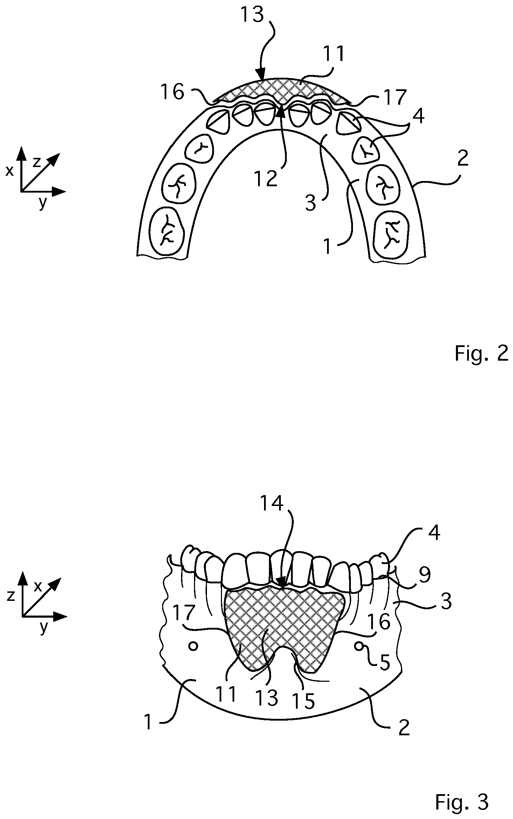

Es ist insbesondere in den Figuren 2 und 3 zu erkennen, dass das Transplant 11 im vorderen Bereich des Unterkiefers an diesem befestigt ist. Das Transplantat 11 weist eine Innenfläche 12 auf, die dem Kieferbogen im Bereich des Knochendefektes entspricht. Mit seiner Innenfläche 12 liegt das Transplantat 11 an dem Kieferknochen 2 an. Das Transplantat 11 weist ferner eine Labialfläche 13 auf, die glatt und abgerundet ist. Das Transplantat 11 weist eine Oberkante 14 und eine Unterkante 15 auf. Die Ausdehnung des Transplantates zwischen seiner Innenfläche 12 und seiner Labialfläche 13 ist seine Dicke (Dickenrichtung x). Diese Dicke sollte im dicksten Abschnitt des Transplantats 11 zwischen 3 und 4 mm liegen. Die Ausdehnung des Transplantates 11 zwischen seiner Unterkante 15 und seiner Oberkante 14 ist seine Höhe (Höhenrichtung z). Die Oberkante ist dabei um einen Versatz v von 1 bis 3 mm in Höhenrichtung z von der Zahnschmelzgrenze 9 beabstandet. Es ist in Fig. 1 zu erkennen, dass sich die Oberkante 14 des Transplantats 11 unterhalb der Zahnschmelzgrenze 9 befindet. Das Transplantat 11 erstreckt sich von der Oberkante 14 nach unten in Richtung des Kinns bis zu seiner Unterkante 15, die von der Unterkante 10 des Kieferkochens 2 beabstandet ist. Schließlich weist das Transplantat 11 eine erste Außenkante 16 und eine dieser gegenüberliegende, zweite Außenkante 17 auf, die sich jeweils von der Oberkante 14 bis zur Unterkante 15 des Transplantats 11 erstrecken. Die Ausdehnung des Transplantates 11 zwischen seiner ersten Außenkante 16 und seiner zweiten Außenkante 17 ist seine Breite (Breitenrichtung y). Die Breite des Transplantats 11 hängt von der Breite des Knochendefektes ab.

Das Transplantat 11 kann Löcher (nicht gezeigt) aufweisen, durch die Schrauben geführt sind, mit deren Hilfe das Transplantat 11 am Kieferknochen 2 befestigt ist. Es ist in Fig. 3 zu erkennen, dass das Transplantat 11 so ausgebildet ist, dass es Nervenenden, die an den Foramina mentale 5 liegen, nicht bedeckt.

Das Transplantat 11 entspricht dem virtuellen Transplantatmodell mit Ausnahme der Tatsache, dass das virtuelle Transplantatmodell nur ein virtuelles Konstrukt ist, während das Transplantat 11 die Verwirklichung des virtuellen Transplantatmodells physisch existiert.

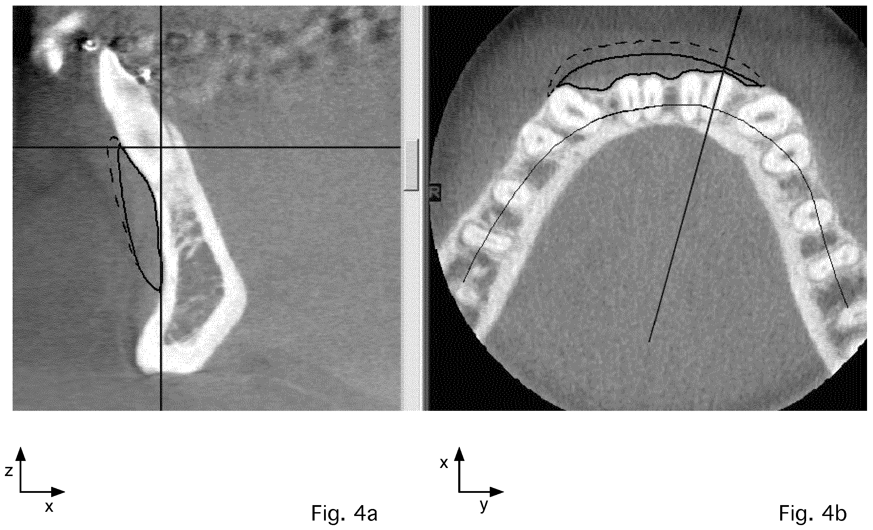

Die Figuren 4a und 4b zeigen Röntgenaufnahmen eines Unterkiefers einen Patienten. Fig. 4a zeigt dabei eine Ansicht, die der Fig. 1 gezeigten Querschnittsdarstellung entspricht. Fig. 4b zeigt eine Ansicht, die der in Fig. 2 gezeigten Draufsicht entspricht. Es ist in Fig. 4a zu erkennen, dass die Oberkante des erfindungsgemäßen Transplantats um einen Versatz v von 1 bis 3 mm in Höhenrichtung z von der Zahnschmelzgrenze beabstandet ist. Damit weist das erfindungsgemäße Transplantat (durchgehende Linie in Fig. 4) eine geringere Ausdehnung als ein Transplantat nach dem Stand der Technik (gestrichelte Linie in Fig. 4) in Höhenrichtung auf. Es insbesondere in Fig. 4b, aber auch Fig. 2 zu erkennen, dass das erfindungsgemäße Transplantat in Dickenrichtung x eine geringere Ausdehnung aufweist. Das Transplantat weist im dicksten Bereich eine Stärke von 3 bis 4 mm auf. Es füllt daher den Knochendefekt auf, ohne in labialer Richtung (x-Achse) wie das Transplantat nach dem Stand der Technik über den Kiefer überzustehen. Dies ist auf den Versatz v zurückzuführen.

### Beispiel zur Anwendung eines erfindungsgemäßen Transplantates

Das erfindungsgemäße Transplantat kann mittels eines vierstufigen Verfahrens einem Patienten implantiert werden.

### Stufe 1: Vorbereitungsphase

Für Patienten mit einem dünnen Biotyp von Gingiva ist eine Augmentation des Weichgewebes notwendig, so dass die Mindestbreite der keratinisierten Gingiva 3 mm beträgt. Erst nach Einheilung und Ausreifung von neuem Gewebe, ca. nach 3 Monaten, wird eine zweite Stufe des Verfahrens geplant und durchgeführt.

### Stufe 2: Herstellung des erfindungsgemäßen Transplantats

Das Transplantat wird unter Anwendung des erfindungsgemäßen Verfahrens hergestellt (siehe Schritte (a) bis (d) des erfindungsgemäßen Verfahrens). Dabei kann das Transplantat aus Eigenknochen des Patienten mittels Fräsen hergestellt werden. Das hergestellte Transplantat kann dann als steriler Block an die Zahnarztpraxis des jeweiligen Patienten zur Implantation mittels eines operativen Eingriffs gesendet werden.

### Stufe 3: Implantation

Das chirurgische Verfahren beginnt mit der Vorbereitung des Operationsfeldes. Ein Mukoperiostlappen in der Region, in der das Transplantat angeordnet werden soll, wird vorbereitet. Um die Blutversorgung in der Transplantationsregion zu verbessern, wird eine Perforation der äußeren Schicht des Patientenknochens durchgeführt. Nach dem die Empfängerregion vorbereitet wurde, wird das gefräste Knochentransplantat übertragen und dort fixiert. Es können zwei Schrauben verwendet werden, eine auf der rechten und eine auf der linken Seite des Transplantats. Der Bereich zwischen dem Knochendefekt und dem Transplantat wird mit allogenen Knochenresten gefüllt. Das Transplantat wird mit einer resorbierbaren Kollagenmembran bedeckt und mit resorbierbaren Fäden vernäht. Nach Mobilisierung des Periostlappens wird die Papille neu positioniert und vernäht. Resorbierbares Nähmaterial eignet sich dafür am besten.

### Stufe 4: Kieferorthopädische Therapie

Vier Wochen nach der Implantation, bevor das Transplantat vollständig einheilt und umgebaut ist, wird die kieferorthopädische Therapie begonnen. Die in diese Region bewegten Zähnen fördern dann den Knochenumbau und sorgen für einen dauerhaften Knochenerhalt.

### Zitierte Literatur

1. Diedrich P. Probleme und Risiken bei der Bewegung von Unterkieferfrontzähnen. Fortschritte der Kieferorthopädie 1995, 56, 148-156
2. Jung SY, Shin SY, Lee KH et al. Analysis of mandibular structure using 3D facial computed tomography. Otolaryngology-head and neck surgery: official journal of American Academy of Otolaryngology-Head and Neck Surgery 2014; 151: 760-764.
3. Batenhorst, K. F., Bowers G. M., Williams I. E.: Tissue changes resulting from facial tipping and extrusion in monkeys, J. Periodont. 45 (1974), 660.
4. Edwards, J. G.: A study of the anterior portion of the palate as it relates to orthodontic therapy. Amer. J. Orthodont. 69 (1976), 249.
5. Engelking, C., Zachrisson B. U.: Die Auswirkung der Schneidezahnretraktion auf das Parodontium von Affen nach vorausgegangener Protrusion durch die Kortikalis. Inf. Orthodont. Kieferorthop. 2 (1983), 127.
6. Karring, T., Nyman S., Thilander B., Magnusson I,: Bone regeneration in orthodontically produced alveolar bone dehiscences. J. periodont. Res. 17 (1982), 309.
7. Steiner, G. G., Pearson J. K., Alnamo J.: Changas of the marginal periodontium as a result of labial tooth movements in monkeys. J. Periodont. 52 (1981), 314.
8. Wennström, J., Lindhe J., Sinclair F., Thilander B.: Same periodontal tissue reactions to orthodontic tooth movement in monkeys. J. olin. Periodont. 14 (1987), 121,
9. Ärtun, J., O. Krogstad: Periodental status of mandibular incisors following excessive proclination. Amer. J. Orthodont. dentofac. Orthop. 91 (1987), 225.
10. Cootoam, G. W., Behrents R. G., Bissada N. F.: The width of keratinezed gingiva during orthodontic treatment: its significance and impact on periodontal status. J. Periodont. 52 (1981), 307.
11. Mulie, R. M., Ten Hoeve A.: The Iimitation of tooth movement within the symphysis studied with laminagraphy and standardized occlusal films. J. elin. Orthodont. 10 (1976), 882.
12. Ten Hoeve, A., Mulie R. M.: The effect of antero-postero incisor repositioning an the palatal cortex as studied with laminagraphy. J. clin. Orthodont. 10 (1976), 804.
13. Rafiuddin S, Yg PK, Biswas S, Prabhu SS, Bm C, Mp R.: Iatrogenic Damage to the Periodontium Caused by Orthodontic Treatment Procedures: An Overview. Open Dent J. 2015 Jun 26; 9: 228-34
14. Schwenzer N., Ehrenfeld M.: Zahn-Mund-Kiefer-Heilkunde. 5 Bände, Band 3: Zahnärztliche Chirurgie. Thieme Verlag, Stuttgart 2000
15. Fanghänel J, Proff P, Dietze S, Bayerlein T, Mack F, Gedrange T.: The morphological and clinical relevance of mandibular and maxillary bone structures for implantation. Folia Morphol (Warsz). 2006 Feb; 65 (1): 49-53.
16. Patent Number: EP 1 066 838 A1; Bone Tissue Regenerating Composition
17. Patent Number: EP 1 862 143 A1; Method for the guided regeneration of bone and/or periodontal tissues in the medical surgical and dental field and device thus obtainable
18. Patent Number: RU2421166 Method of gum recession removal
19. Patent Number: RU2241395 Method for plasty of mandibular segmentary defects
20. Babbush CA. The use of a new allograft material for osseous reconstruction associated with dental implants. Implant Dent. 1998; 7 (3): 205-12.
21. Proft P, Bayerlein T, Fanghänel J, Gerike W, Bienengräber V, Gedrange T.: The application of bone graft substitutes tor alveolar ridge preservation after orthodontic extractions and for augmentation of residual cleft defects. Folia Morphol (Warsz). 2006 Feb; 65 (1): 81-3.
22. Shchudlo NA, Borisova IV, Krasnov VV, Dobychina NA.: State of lower alveolar and mental nerves by mandible fractures healing under transbone osteosynthesis. Stomatologiia (Mosk). 2012; 91 (3): 4-6.
23. Owman-Moll P. Orthodontic tooth movement and root resorption with special reference to force magnitude and duration. A clinical and histological investigation in adolescents. Swed Dent J Suppl. 1995; 105: 1-45.
24. Danan M, Zenou S, Bouaziz-Attal AS, Dridi SM. Orthodontic traction of an impacted canine through a synthetic bone substitute. J Clin Orthod. 2004 Jan; 38 (1): 39-44.
25. Ogihara S, Marks MH. Alveolar bone upper growth in furcation area using a combined orthodontic-regenerative therapy: a case report. J Periodontol. 2002 Dec; 73 (12): 1522-7.
26. Gedrange T, Gredes T, Spassow A, Mai R, Alegrini S, Dominiak M, Kunert-Keil C, Hainemann F. Orthodontic tooth movement into jaw regions treated with synthetic bone substitute. Ann Acad Med Stetin. 2010; 56 (2): 80-4.
27. Apostolakis D, Brown JE. The dimensions of the mandibular incisive canal ar'ld its spatial relationship to various anatomical Iandmarks of the mandible: a study using cone beam computed tomography. The Internationaljournal of oral & maxillofacial implants 2013; 28: 117-124.
28. Miller RJ, Edwards WC, Boudet C et al. Maxillofacial anatomy: the mandibular symphysis. The Journal of oral implantology 2011; 37: 745-753.
29. Pires CA, Bissada NF, Becker JJ et al. Mandibular incisive canal: cone beam computed tomography. Clinical im plant dentistry and related research 2012; 14: 67-73.
30. Joss-Vassalli I, Grabenstein C., Topouzelis N., Sculean A., Katsaras C. Kieferorthopädische Therapie und Gingivarezessionen: Inf Orthod Kieferorthop 2011; 43: 263-273.

### Bezugszeichenliste

- 1: Unterkiefer
- 2: Kieferknochen
- 3: Alveolarfortsatz
- 4: Zahn
- 5: Foramen mentale
- 6: Zahnkrone
- 7: Zahnschmelz
- 8: Zahnwurzel
- 9: Zahnschmelzgrenze
- 10: Unterkante

- 11: Transplantat
- 12: Innenfläche
- 13: Labialfläche
- 14: Oberkante
- 15: Unterkante
- 16: erste Außenkante
- 17: zweite Außenkante

## Patentansprüche

1. Verfahren zur Herstellung eines Transplantats (11) zur Beseitigung eines Knochendefektes am Kiefer (1) eines Patienten, umfassend die Schritte:
(a) Anfertigen einer dreidimensionalen Aufnahme des Kiefers (1) des Patienten oder eines Teiles des Kiefers (1), an dem sich der Kieferknochendefekt befindet;
(b) Anfertigen eines virtuellen Modells des Kiefers (1) oder des Teiles des Kiefers (1), an dem sich der Knochendefekt befindet, anhand der dreidimensionalen Aufnahme unter Erhalt eines virtuellen Kiefermodells;
(c) Anfertigen eines virtuellen Modells des Transplantats (11) auf Basis des virtuellen Kiefermodelles unter Erhalt eines virtuellen Transplantatmodells, wobei das virtuelle Transplantatmodell eine Innenfläche (12), die dem Kieferbogen des virtuellen Kiefermodells im Bereich des Knochendefektes entspricht und mit der das virtuelle Transplantatmodell an dem virtuellen Kiefermodell im Bereich des Knochendefektes anliegt, und eine Längskante aufweist, die der Zahnschmelzgrenze zugewandt ist und die um einen Versatz zum Niveau der Zahnschmelzgrenze beabstandet ausgebildet ist; und
(d) Herstellung des Transplantates (11) anhand des virtuellen Transplantatmodells.

2. Verfahren nach Anspruch 1, **dadurch gekennzeichnet, dass** der Versatz zum Niveau der Zahnschmelzgrenze (9) in einem Bereich von 1 mm bis 3 mm liegt.

3. Verfahren nach Anspruch 1 oder Anspruch 2, **dadurch gekennzeichnet, dass** das in Schritt (c) angefertigte virtuelle Transplantatmodell eine Breite aufweist, die der Ausdehnung des Knochendefektes des virtuellen Kiefermodells in Breitenrichtung entspricht.

4. Verfahren nach einem der vorstehenden Ansprüche, **dadurch gekennzeichnet, dass** das in Schritt (c) angefertigte virtuelle Transplantatmodell Löcher aufweist, die im Transplantat (11) die Durchführung von Fixierungsmitteln ermöglichen, wobei die Löcher im virtuellen Transplantatmodell so ausgebildet sind, dass sie jeweils in einem Zahnzwischenraum des virtuellen Kiefermodells liegen.

5. Verfahren nach einem der vorstehenden Ansprüche, **dadurch gekennzeichnet, dass** das in Schritt (c) angefertigte virtuelle Transplantatmodell eine maximale Dicke in einem Bereich von 3 bis 4 mm aufweist.

6. Verfahren nach einem der vorstehenden Ansprüche, **dadurch gekennzeichnet, dass** die Kanten (14, 15, 16, 17) des virtuellen Transplantatmodells abgerundet sind.

7. Verfahren nach einem der vorstehenden Ansprüche, **dadurch gekennzeichnet, dass** die Labialfläche(n) (13) des virtuellen Transplantatmodells glatt sind.

8. Verfahren nach einem der vorstehenden Ansprüche, **dadurch gekennzeichnet, dass** in Schritt (d) zur Herstellung des Transplantates (11) Knochenmaterial des Patienten eingesetzt wird.

9. Verfahren nach einem der vorstehenden Ansprüche, **dadurch gekennzeichnet, dass** in Schritt (a) die dreidimensionale Aufnahme mittels Computertomographie, Magnetresonanztomographie, dentaler Volumentomographie oder anderer 3D-Aufnahmetechniken angefertigt wird.

10. Verfahren nach einem der vorstehenden Ansprüche, **dadurch gekennzeichnet, dass** der Teil des Kiefers (1) der Unterkiefer, der Kieferknochen (2) des Unterkiefers oder der Alveolarfortsatz (3) des Unterkiefers mit dem Alveolarknochen ist.

11. Transplantat zur Beseitigung eines Knochendefektes am Kiefer eines Patienten, **dadurch gekennzeichnet, dass** das Transplantat (11) eine Innenfläche (12), die dem Kieferbogen des Kiefers oder des Teiles davon, der den Knochendefekt aufweist, entspricht, und eine Längskante aufweist, die der Zahnschmelzgrenze zugewandt ist und die um einen Versatz zum Niveau der Zahnschmelzgrenze beabstandet ausgebildet ist.

12. Transplantat nach Anspruch 11, **dadurch gekennzeichnet, dass** es eine Längskante (14) aufweist, die um einen Versatz zum Niveau der Zahnschmelzgrenze (9) beabstandet ausgebildet ist, wobei der Versatz zum Niveau der Zahnschmelzgrenze (9) in einem Bereich von 1 mm bis 3 mm liegt.

13. Transplantat nach Anspruch 11 oder Anspruch 12, **dadurch gekennzeichnet, dass** es eine Breite aufweist, die der Ausdehnung des Knochendefektes des virtuellen Kiefermodells in Breitenrichtung entspricht.

14. Transplantat, hergestellt mittels eines Verfahrens nach einem der Ansprüche 1 bis 10.

15. Virtuelle Anordnung, aufweisend
(i) ein erstes virtuelles Modell eines Kiefers (1) oder des Teiles eines Kiefers, an dem sich ein Knochendefekt befindet; und
(ii) ein zweites virtuelles Modell eines Transplantats (11),
**dadurch gekennzeichnet, dass** das zweite virtuelle Modell eine Innenfläche (12), die dem Kieferbogen des ersten virtuellen Modells entspricht und mit der das zweite virtuelle Modell an dem ersten virtuellen Modell im Bereich des Knochendefektes anliegt, und eine Längskante aufweist, die der Zahnschmelzgrenze zugewandt ist und die um einen Versatz zum Niveau der Zahnschmelzgrenze beabstandet ausgebildet ist.

## Claims

1. A method of preparing a graft (11) for eliminating a bone defect in a patient's jaw (1), comprising the steps of:
(a) taking a three-dimensional image of the patient's jaw (1) or a part of the jaw (1) where the jaw-bone defect is located;
(b) making a virtual model of the jaw (1) or of the part of the jaw (1) where the bone defect is located based on the three-dimensional image to obtain a virtual jaw model;
(c) making a virtual model of the graft (11) based on the virtual jaw model to obtain a virtual graft model, wherein the virtual graft model has an inner surface (12), which corresponds to the jaw arch of the virtual jaw model in the region of the bone defect and with which the virtual graft model is adjacent to the virtual jaw model in the region of the bone defect, and a longitudinal edge that faces the cemento-enamel junction and that is formed spaced by an offset to the level of the cemento-enamel junction; and
(d) producing the graft (11) based on the virtual graft model.

2. The method according to claim 1, **characterized in that** the offset to the level of the cemento-enamel junction (9) is in the range of 1 mm to 3 mm.

3. The method according to claim 1 or claim 2, **characterized in that** the virtual graft model made in step (c) has a width that corresponds to the extent of the bone defect of the virtual jaw model in the width direction.

4. The method according to any of the preceding claims, **characterized in that** the virtual graft model made in step (c) has holes that enable passing through fixatives in the graft (11), wherein the holes are formed in the virtual graft model such that they each lie within an interdentium of the virtual jaw model.

5. The method according to any of the preceding claims, **characterized in that** the virtual graft model made in step (c) has a maximum thickness in a range of 3 to 4 mm.

6. The method according to any of the preceding claims, **characterized in that** the edges (14, 15, 16, 17) of the virtual graft model are rounded.

7. The method according to any of the preceding claims, **characterized in that** the labial surface(s) (13) of the virtual graft model are even.

8. The method according to any of the preceding claims, **characterized in that** in step (d) of preparing the graft (11) bone material of the patient is used.

9. The method according to any of the preceding claims, **characterized in that** in step (a) the three-dimensional image is taken by means of computerized tomography, magnetic resonance tomography, dental volume tomography, or other 3D radiographic techniques.

10. The method according to any of the preceding claims, **characterized in that** the part of the jaw (1) is the mandible, the jaw-bone (2) of the mandible, or the alveolar process (3) of the mandible with the alveolar bone.

11. A graft for eliminating a bone defect in a patient's jaw, **characterized in that** the graft (11) has an inner surface (12) corresponding to the jaw arch of the jaw or of the part thereof having the bone defect, and a longitudinal edge that faces the cemento-enamel junction and that is formed spaced by an offset to the level of the cemento-enamel junction.

12. The graft according to claim 11, **characterized in that** it has a longitudinal edge (14) formed spaced by an offset to the level of the cemento-enamel junction (9), wherein the offset to the level of the cemento-enamel junction (9) is in a range of 1 mm to 3 mm.

13. The graft according to claim 11 or claim 12, **characterized in that** it has a width that corresponds to the extent of the bone defect of the virtual jaw model in the width direction.

14. A graft produced by means of a method according to any of claims 1 to 10.

15. A virtual arrangement having
(i) a first virtual model of a jaw (1) or of the part of a jaw where the bone defect is located; and
(ii) a second virtual model of a graft (11),
**characterized in that** the second virtual model has an inner surface (12), which corresponds to the jaw arch of the first virtual model and with which the second virtual model is adjacent to the first virtual model in the region of the bone defect, and a longitudinal edge that faces the cemento-enamel junction and that is formed spaced by an offset to the level of the cemento-enamel junction.

## Revendications

1. Procédé pour la fabrication d'un greffon (11) pour la suppression d'une défectuosité osseuse sur la mâchoire (1) d'un patient, comprenant les étapes suivantes:
(a) Confection d'une vue tridimensionnelle de la mâchoire (1) du patient ou d'une partie de la mâchoire (1) dans laquelle se trouve la défectuosité osseuse;
(b) Confection d'un modèle virtuel de la mâchoire (1) ou d'une partie de la mâchoire dans laquelle se trouve la défectuosité osseuse, à partir de la vue tridimensionnelle en conservant un modèle virtuel de la mâchoire;
(c) Confection d'un modèle virtuel du greffon (11) sur la base du modèle virtuel de la mâchoire en conservant un modèle virtuel du greffon, celui-ci présentant une surface intérieure (12) correspondant, dans la zone de défectuosité osseuse, à la courbure de la mâchoire du modèle virtuel de la mâchoire, avec laquelle le modèle virtuel de greffon s'ajuste au modèle virtuel de la mâchoire dans la zone de défectuosité osseuse, et une arête longitudinale qui fait face à la limite de l'émail dentaire et forme un décalage espacé par rapport au niveau de la limite de l'émail dentaire; et
(d) fabrication du greffon (11) selon le modèle du greffon virtuel.

2. Procédé selon la revendication 1, **caractérisé par le fait que** le décalage par rapport au niveau de la limite de l'émail dentaire (9) se trouve dans une zone de 1 mm à 3 mm.

3. Procédé selon la revendication 1 ou la revendication 2, **caractérisé par le fait que** le modèle de greffon virtuel confectionné dans l'étape (c) présente une largeur qui correspond en latitude à l'étendue de la défectuosité osseuse du modèle de mâchoire virtuel.

4. Procédé selon une des revendications précédentes, **caractérisé par le fait que** le modèle de greffon virtuel confectionné dans l'étape (c) présente des trous qui permettent la réalisation de moyens de fixation dans le greffon (11), lesdits trous étant réalisés dans le modèle de greffon virtuel de façon à ce que chacun d'eux se trouve dans un intervalle de dents du modèle de mâchoire virtuel.

5. Procédé selon une des revendications précédentes, **caractérisé par le fait que** le modèle de greffon virtuel confectionné dans l'étape (c) présente une épaisseur maximum située dans une zone de 3 à 4 mm.

6. Procédé selon une des revendications précédentes, **caractérisé par le fait que** les arêtes (14, 15, 16, 17) du modèle de greffon virtuel sont arrondies.

7. Procédé selon une des revendications précédentes, **caractérisé par le fait que** la/les surface(s) labiale(s) (13) du modèle de greffon virtuel est/sont lisse(s).

8. Procédé selon une des revendications précédentes, **caractérisé par le fait que**, dans l'étape (d), du matériau osseux du patient est utilisé pour la fabrication du greffon (11).

9. Procédé selon une des revendications précédentes, **caractérisé par le fait que**, dans l'étape (a) la vue tridimensionnelle est effectuée par tomodensitométrie, tomographie par résonnance magnétique, tomographie dentaire volumique ou autres techniques d'imagerie tridimensionnelle.

10. Procédé selon une des revendications précédentes, **caractérisé par le fait que** la partie de la mâchoire (1) est soit la mandibule, l'os de la mâchoire (2) de la mandibule ou le rebord alvéolaire (3) de la mandibule avec l'os alvéolaire.

11. Greffon pour la suppression d'une défectuosité osseuse sur la mâchoire d'un patient, **caractérisé par le fait que** le greffon (11) présente une surface intérieure (12) qui correspond à la courbure de la mâchoire, ou d'une partie de celui-ci, présentant une défectuosité osseuse, et une arête longitudinale qui fait face à la limite de l'émail dentaire et forme un décalage espacé par rapport au niveau de la limite de l'émail dentaire.

12. Greffon selon la revendication (11) **caractérisé par le fait qu'**il présente une arête longitudinale (14) qui est configurée pour former un décalage espacé par rapport au niveau de la limite de l'émail dentaire (9), ledit décalage par rapport au niveau de la limite de l'émail dentaire se trouvant dans une zone de 1 mm à 3 mm.

13. Greffon selon la revendication 11 ou la revendication 12, **caractérisé par le fait qu'**il présente une largeur correspondant en latitude à l'étendue de la défectuosité osseuse du modèle de mâchoire virtuel.

14. Greffon fabriqué par un procédé selon une des revendications 1 à 10.

15. Configuration virtuelle présentant
(i) un premier modèle virtuel d'une mâchoire (1) ou d'une partie d'une mâchoire sur lequel se trouve une défectuosité osseuse; et
(ii) un deuxième modèle virtuel d'un greffon (11),
**caractérisé par le fait que** le deuxième modèle virtuel présente une surface intérieure (12) qui correspond à la courbure de la mâchoire du premier modèle et avec laquelle le deuxième modèle virtuel s'ajuste au premier modèle virtuel dans la zone de la défectuosité osseuse, et une arête longitudinale faisant face à la limite de l'émail dentaire et configuré pour former un décalage espacé par rapport au niveau de la limite de l'émail dentaire.
